# EUROPEAN PATENT SPECIFICATION

(11) **EP 0 285 219 B1**
(45) Date of publication and mention of the grant of the patent: **11.09.1996**
(21) Application number: 88200587.9
(22) Date of filing: 29.03.1988
(51) Int. Cl.: C07D 241/04, C07D 401/12, A61K 31/495

(54) **Method of improving sleep**
Methode zur Verbesserung des Schlafes
Méthode pour améliorer le sommeil

(30) Priority: 01.04.1987 US 34129
(43) Date of publication of application: 05.10.1988
(60) Divisional application: 96200236.6
(73) Proprietor: JANSSEN PHARMACEUTICA N.V., B-2340 Beerse (BE)
(72) Inventor: Van Daele, Georges Henri Paul, B-2300 Turnhout (BE); Vlaeminck, Freddy François, B-2418 Lille (BE); Verdonck, Marc Gustaaf Celine, B-2300 Turnhout (BE)

(56) References cited:
- EP-A- 0 068 544
- PSYCHOPHARMACOLOGY, vol. 91, no. 4, 3rd April 1987, pages 434-439, Springer-Verlag; A. WAUQUIER et al.: "Sleep improvement in dogs after oral administration of mioflazine, a nucleoside transport inhibitor"

## Description

A new method of treating sleep disorders is generally considered an important goal to achieve. Up until now, quite a number of preparations are known which effect sleep, said preparations containing usually as active ingredient hypnotics such as, benzodiazepines, barbiturates and the like.

The present invention is concerned with the use for the manufacture of a medicament for improving sleep and treating sleep disorders of particular N-aryl-piperazinealkanamide derivatives.

Some of the N-aryl-piperazinealkanamide derivatives of the present invention are known from the Eur. Pat. No. 0,068,644, and were taught to be useful for protecting the heart from myocardial injury caused by ischaemia, anoxia or hypoxia.

Further some N-aryl-piperazinealkanamide derivatives bearing an alkyl substituent on the piperazine moiety are described in U.S. Pat. No. 3,267,104 as coronary vasodilators, as local anaesthetics, as central nervous system stimulating agents, and as anticarrageenin agents.

However, most of the said N-aryl-piperazinealkanamide derivatives are novel and have especially been developed to be used as active substances in the method of the present invention.

The present invention is concerned with the use for the manufacture of a medicament for improving sleep and treating sleap disorders in warm-blooded animals of a piperazine derivative having the formula: 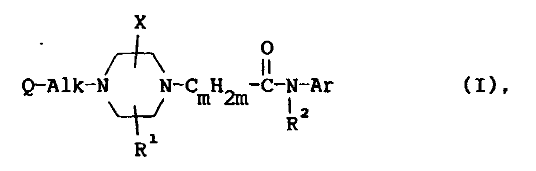 the stereochemically isomeric forms and the pharmaceutically acceptable acid addition salts thereof, wherein:
R¹ is hydrogen or C₁₋₆alkyl;
X is C₁₋₆alkyl, hydroxyC₁₋₆alkyl, C₁₋₆alkyloxyC₁₋₆alkyl, aminocarbonyl, mono- and di(C₁₋₆alkyl)aminocarbonyl, carboxyl, C₁₋₆alkyloxycarbonyl, (aminocarbonyl)C₁₋₆alkyl, [mono- and di(C₁₋₆alkyl)aminocarbonyl]C₁₋₆alkyl, carboxylC₁₋₆alkyl, (C₁₋₆alkyloxycarbonyl)C₁₋₆alkyl or (hydroxyC₁₋₆alkyl)aminocarbonyl;
m is the integer 1 or 2;
R² is hydrogen or C₁₋₆alkyl;
Ar is phenyl, optionally substituted with up to 3 substituents each independently selected from the group consisting of hydroxy, C₁₋₆alkyl, C₁₋₆alkyloxy, halo, trifluoromethyl, C₁₋₆alkylcarbonyl, mono- and di(C₁₋₆alkyl)aminocarbonyl, aminocarbonyl, C₁₋₆alkyloxycarbonyl, nitro, cyano, amino, amino-methyl, mono- and di(C₁₋₆alkyl)amino, (C₁₋₆alkylcarbonyl)amino, (aminocarbonyl)amino and phenylmethoxy; pyridinyl, optionally substituted with up to three substituents independently selected from halo and C₁₋₆alkyl; pyrazolyl, substituted with up to three substituents independently selected from C₁₋₆alkyl; or a radical of formula 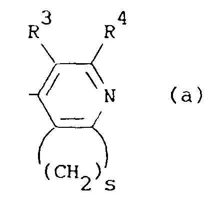 wherein R³ and R⁴ are each independently selected from the group consisting of halo, C₁₋₆alkyl, hydroxy and C₁₋₆alkyloxy and s is the integer 3, 4 or 5;
Alk is a C₁₋₆alkanediyl radical or a C₃₋₆alkenediyl radical, said C₁₋₆alkanediyl radical being optionally substituted with a hydroxy- or a C₁₋₆alkyl radical; and
Q is aryl, aryloxy, diarylmethoxy, 2,2-diarylethenyl, diarylmethylcarbonyl, arylcarbonyl, mono- and diarylaminocarbonyl, diarylmethyl or arylamino, the amino moiety in said arylamino being optionally substituted with an aryl-, an arylcarbonyl-, a C₁₋₆alkylcarbonyl-, an arylsulfonyl- or a C₁₋₆alkylsulfonyl-radical;
wherein aryl is phenyl, substituted phenyl, naphthalenyl, thienyl or pyridinyl, said substituted phenyl having from 1 to 2 substituents, each independently selected from the group consisting of halo and C₁₋₆alkyloxy.

In the foregoing definitions the term halo is generic to fluoro, chloro, bromo and iodo, with fluoro being preferred; the term "C₁₋₆alkyl" is meant to include straight and branched saturated hydrocarbon radicals having from 1 to 6 carbon atoms such as, for example, methyl, ethyl, 1-methylethyl, 1,1'-dimethylethyl, propyl, butyl, pentyl and the like;
"C₁₋₆ alkanediyl" is meant to include bivalent straight or branch chained alkanediyl radicals having from 1 to 6 carbon atoms; and "C₃₋₆ alkenediyl" is meant to include bivalent straight and branch chained hydrocarbon radicals containing one double bond and having from 3 to 6 carbon atoms and when a C₃₋₆alkenediyl is substituted on a heteroatom, then the carbon atom of said C₃₋₆alkenediyl connected to said heteroatom preferably is saturated.

It is to be understood that the compounds of formula (I) may exist in hydrated or in solvent addition forms and that the invention includes all such forms.

Preferred compounds of formula (I) for use in the preparation of a medicament according to the present invention are those compounds of formula (I) wherein R¹ and R² are both hydrogen; m is 1; and X is C₁₋₆ alkyl, hydroxyC₁₋₆alkyl, aminocarbonyl or mono- and di(C₁₋₆alkyl)aminocarbonyl.

Particularly preferred compounds are those preferred compounds of formula (I) wherein Q is diarylmethoxy, 2,2-diarylethenyl, diarylaminocarbonyl, diarylmethyl or arylamino, the amino moiety in said arylamino being substituted with an aryl- or an arylcarbonyl radical; and said aryl being phenyl or substituted phenyl.

Especially preferred compounds are those particularly preferred compounds of formula (I) wherein Q-Alk- is 5,5-di(halophenyl)pentenyl or 5,5-di(halophenyl)pentyl.

An interesting subgroup of compounds of formula (I) to be used in the preparation of a medicament according to the present invention comprises those compounds, preferred or particularly preferred compounds wherein Alk is C₃₋₅alkanediyl, with those compounds having five atoms between the piperazine moiety and the aryl or diaryl moiety in Q constituting a particularly interesting subgroup.

Most preferred compounds are selected from the group consisting of 2-(aminocarbonyl)-N-(4-amino-2,6-dichlorophenyl)-4-[5,5-bis(4-fluorophenyl)pentyl]-1-piperazineacetamide, the pharmaceutically acceptable acid addition salts and the possible stereochemically isomeric forms thereof.

Some compounds of formula (I) are known from U.S. Patent No. 3,267,104 and from the Eur. Pat. No. 68,544, while others are new. The compounds of formula (I) can be prepared following the methods described in the aforementioned patents or by analogous methods.

The compounds of formula (I) can be used as such or in their acid-addition salt form. The latter can conveniently be obtained by treating the base-form with appropriate acids, such as, for example, inorganic acids, such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (z)-2-butenedioic, (E)-2-butenedioic, 2-hydroxybutanedioic, 2,3-dihydroxybutanedioic, 2-hydroxy-1,2,3- propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

The compounds of formula (I) and some of the intermediates in this invention have one or more asymmetric carbon atoms in their structure. Each of these chiral centers may be present in a R- and a S-configuration, this R- and S-notation being in correspondence with the rules described by R.S. Cahn, C. Ingold and V. Prelog in Angew. Chem., Int. Ed. Engl., 5, 385, 511 (1966).

The compounds of formula (I) containing an alkene moiety may be present in a "E" or "Z" form, said E- and Z- notation having the meanings described in J. Org. Chem., 35, 2849-2868 (1970).

Pure stereochemically isomeric forms of the compounds of formula (I) may be obtained by the application of art-known procedures.
Diastereoisomers may be separated by physical separation methods such as selective crystallization and chromatographic techniques, e.g., counter current distribution, and enantiomers may be separated from each other by the selective crystallization of their diastereomeric salts with optically active acids.

Pure stereochemically isomeric forms may also be derived from the corresponding pure stereochemically isomeric forms of the appropriate starting materials, provided that the reaction occurs stereospecifically.

It is evident that the cis and trans diastereomeric racemates may be further resolved into their optical isomers, cis(+), cis(-), trans(+) and trans(-) by the application of methodologies known to those skilled in the art.

The use of the compounds of formula (I), the pharmaceutically acceptable acid-addition salts and stereochemically isomeric forms thereof in the preparation of a medicament according to the present invention is based on their useful sleep improving properties. More particularly, they increase the total sleep, primarily through enhancement of slow wave sleep and decrease of wakening. This property is clearly evidenced by the results obtained in the "slow-wave Sleep in Dogs"-test. By virtue of their ability to improve sleep it is evident that the compounds of the present invention are useful for improving sleep in warm-blooded animals suffering from sleep disorders.

An additional advantage of the medicaments of the present invention comprises the fact that the compounds of formula (I) show the aforementioned sleep improving properties upon oral administration. Apart from their sleep-improving properties, the compounds of the present invention also possess the same useful pharmacological properties of the compounds of the Publ. Eur. Pat. Appl. No. 68,644 and more particularly of the preferred compound thereof, i.e., 3-(aminocarbonyl)-4-[4,4-bis(4-fluorophenyl)butyl]-N-(2,6-dichlorophenyl)-1-piperazineacetamide which generically is designated as mioflazine. Said useful pharmacological properties are described in the mentioned Publ. Eur. Pat. Appl. No. 68,644 and e.g. in Cardiovascular Research, 18, 528-537 (1984), in Cardiovascular Research, 20, 658-664 (1986), and more particularly comprise the capability to ameliorate the blood perfusion of the muscular tissues of the heart, the protection of the heart from myocardial injury, the protection against myocardial calcium-over-load and the inhibition of nucleoside transport.

The compounds of formula (I) may be used as such in the manufacture of a medicament according to the present invention or as suitable pharmaceutical compositions.

To prepare such pharmaceutical compositions, an effective amount of the compound of formula (I), in base or acid-addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not introduce a significant deletorious effect on the skin. said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions. It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoon-fuls and the like, and segregated multiples thereof.

Those of skill in the pertinent art could easily determine what could be an effective sleep-improving amount from the results presented hereinafter. In general it is contemplated that an effective amount would be from 0.001 mg/kg to 100 mg/kg body weight, and more preferably from 0.01 mg/kg to 10 mg/kg body weight.

The following example is intended to illustrate the scope of the invention.

### EXPERIMENTAL PART

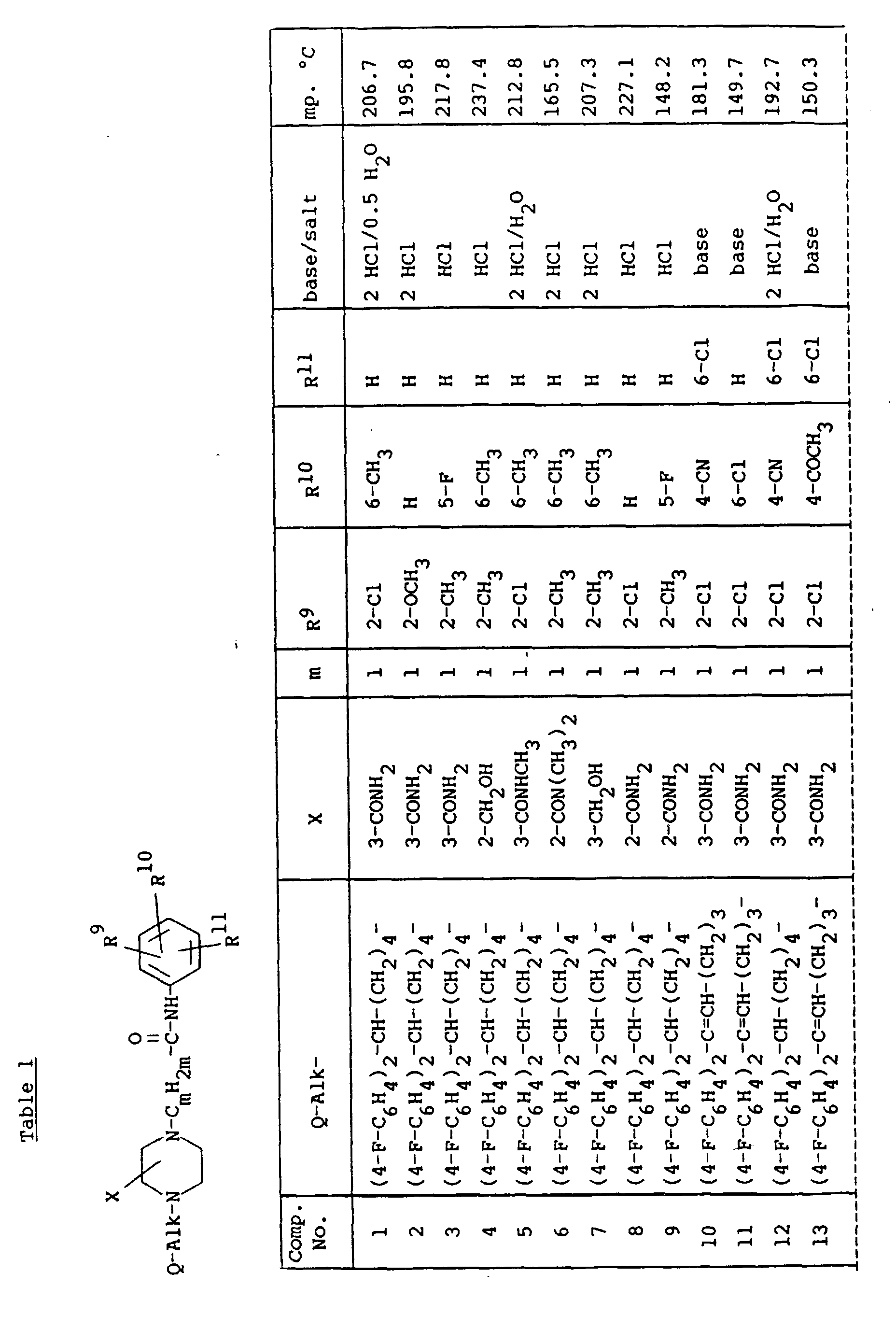

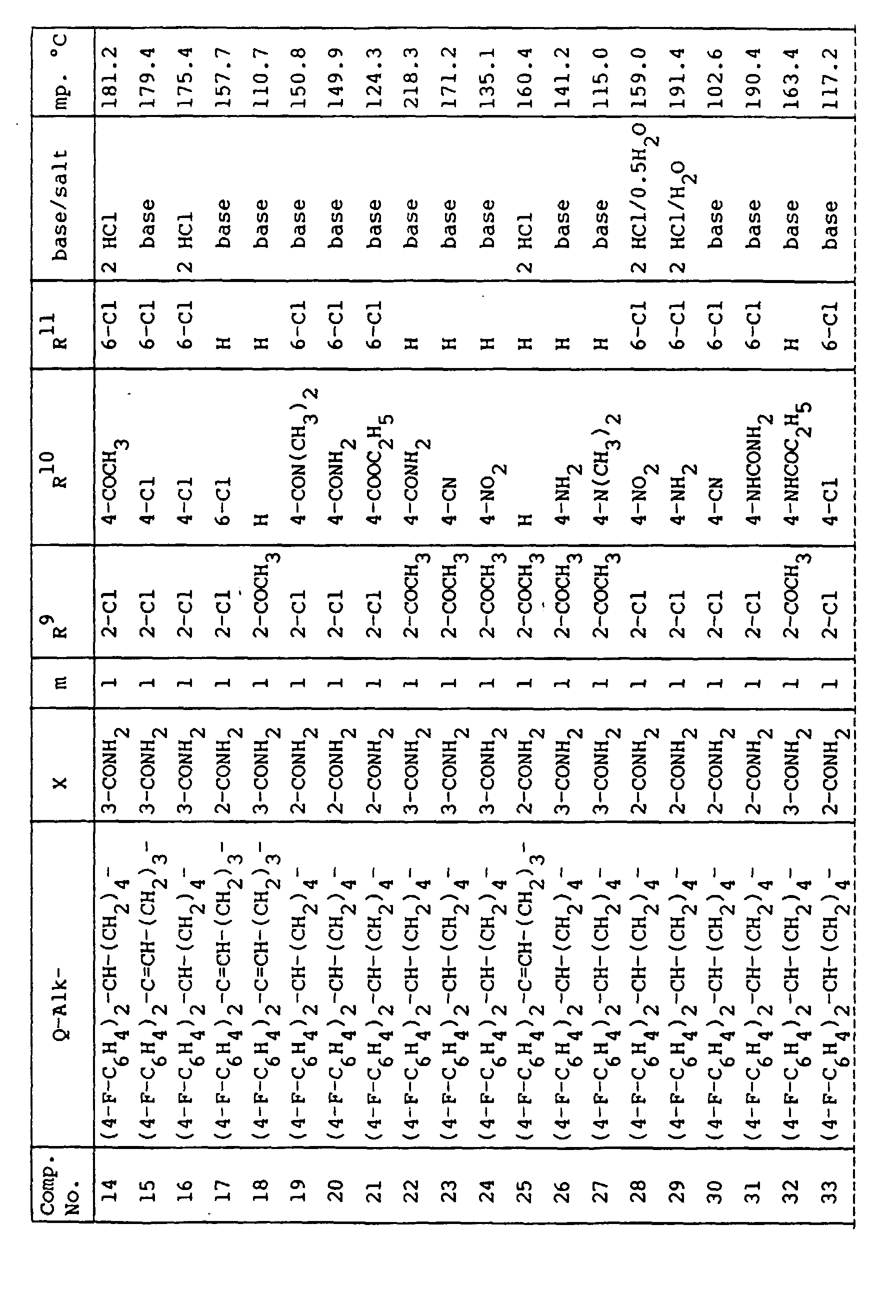

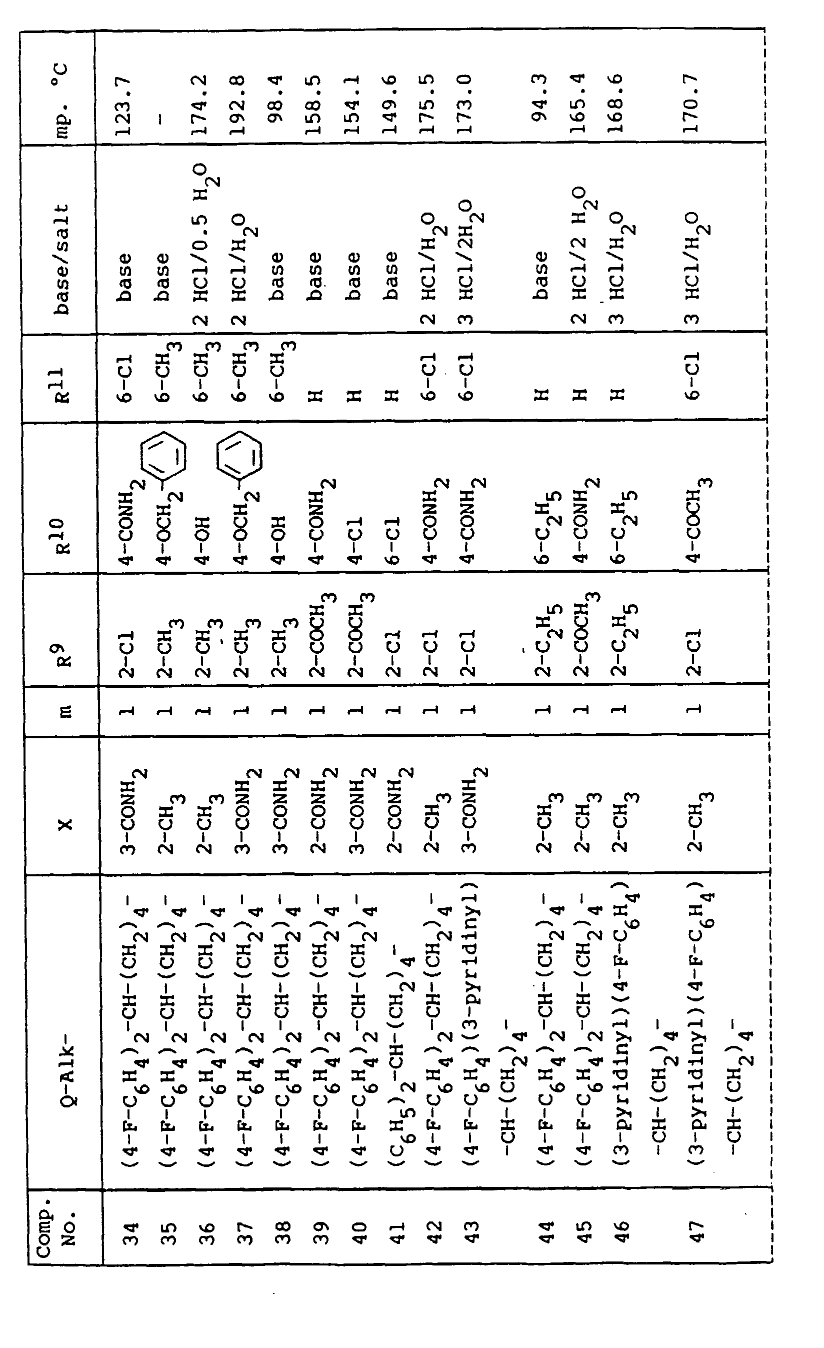

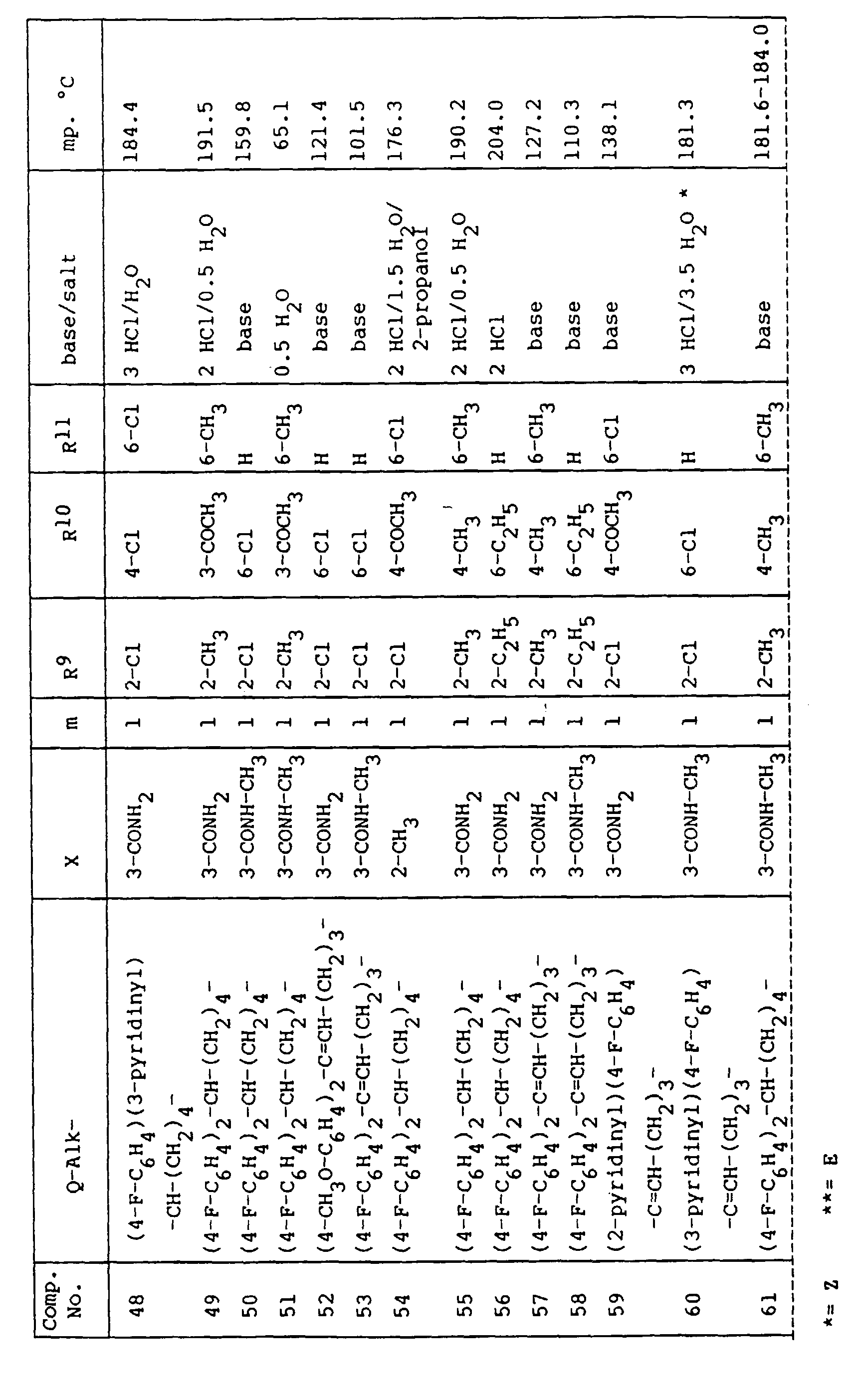

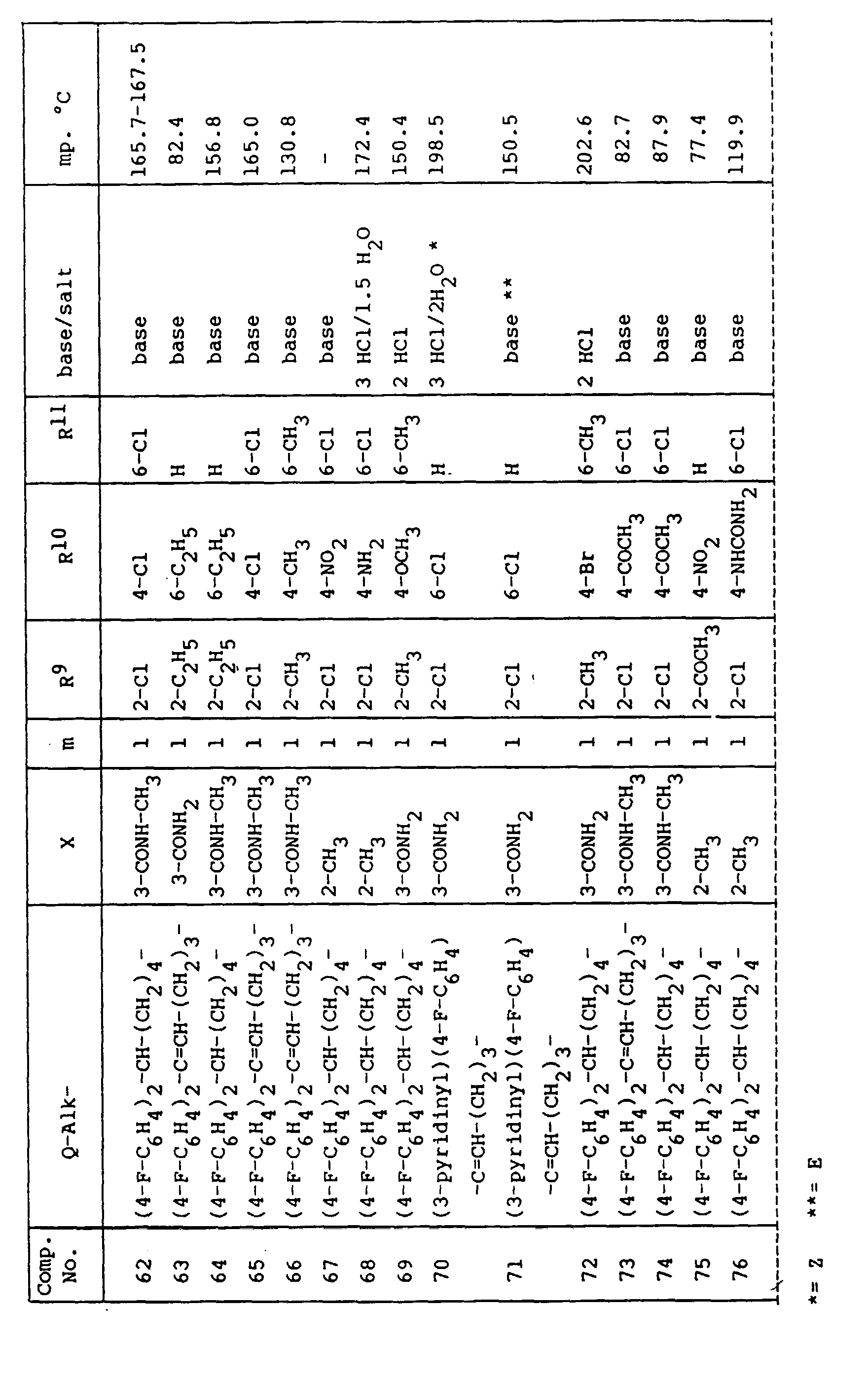

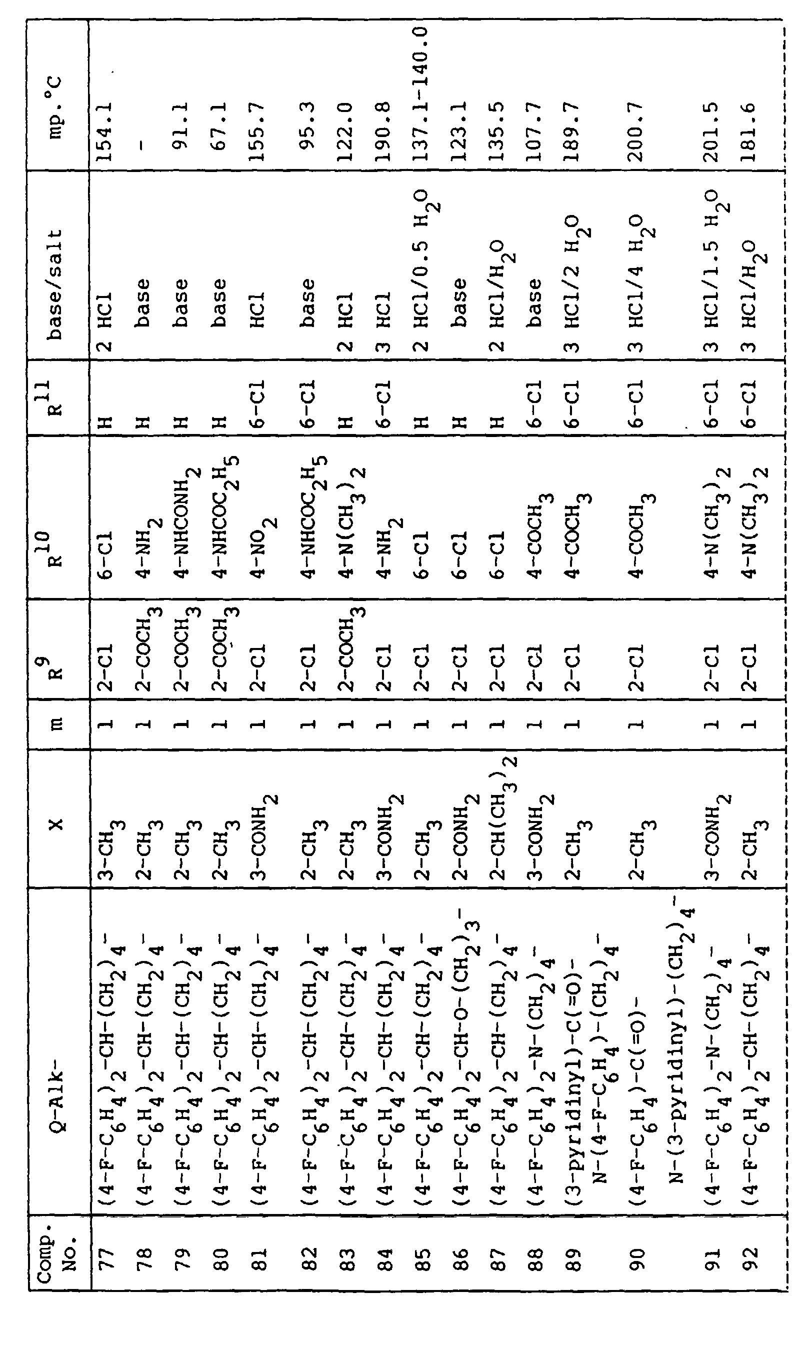

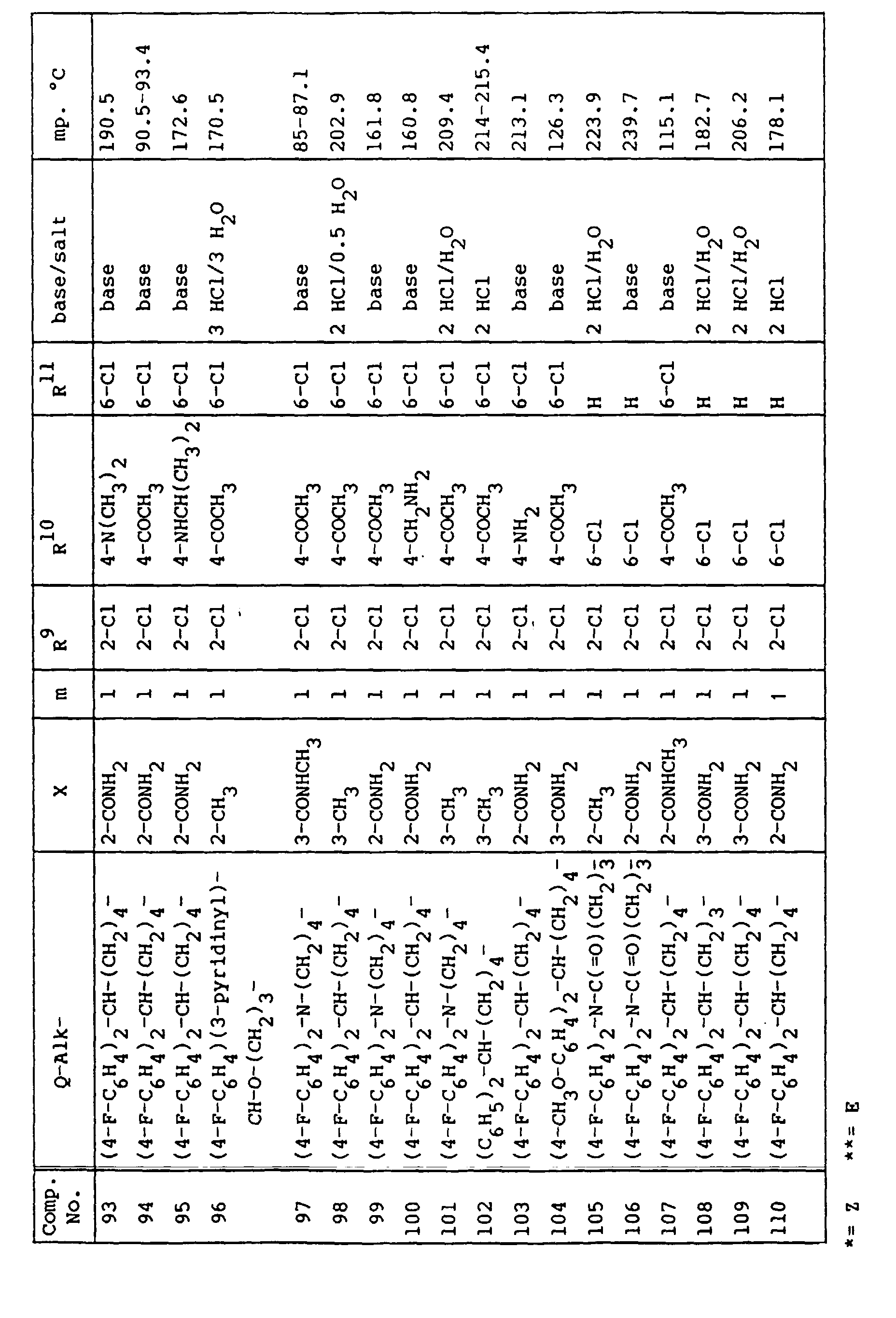

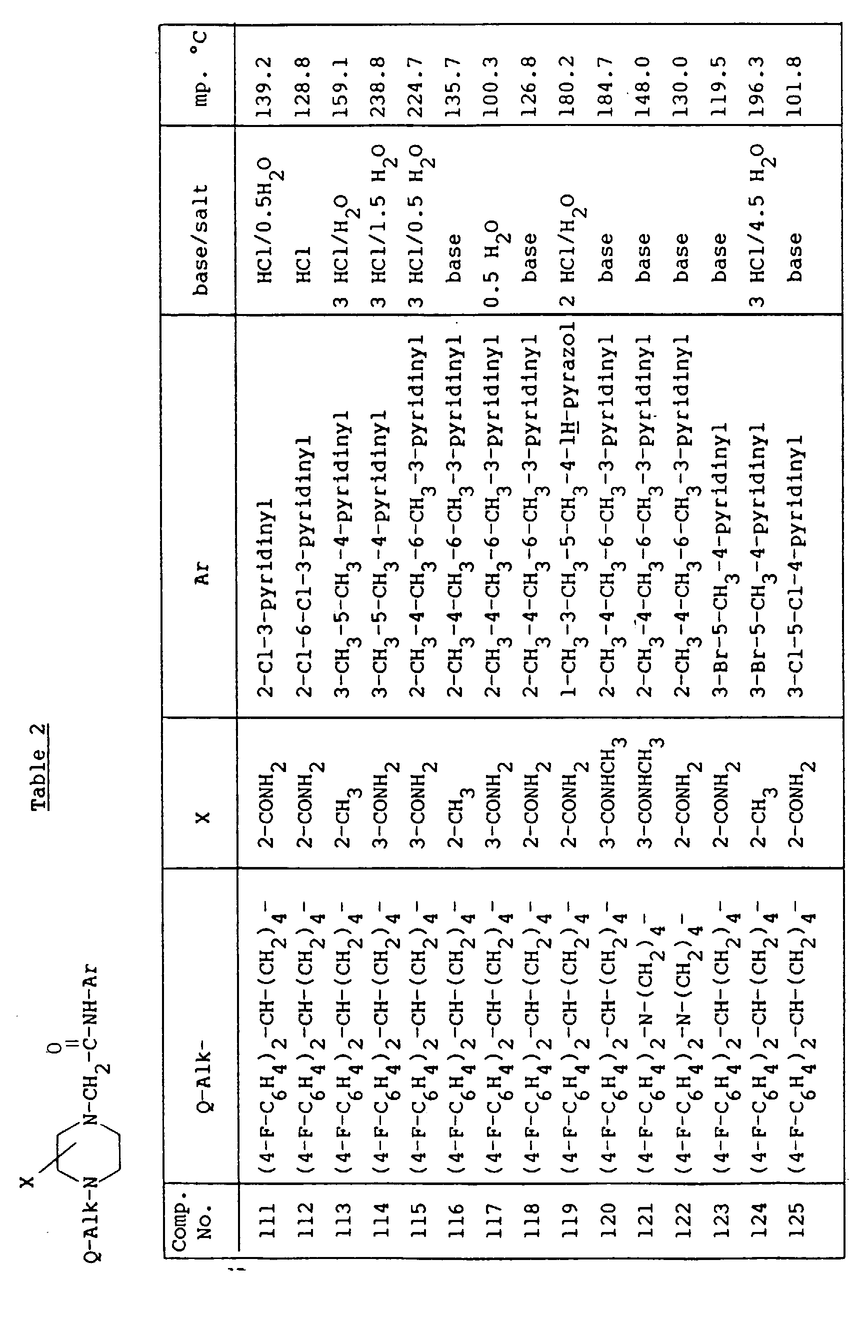

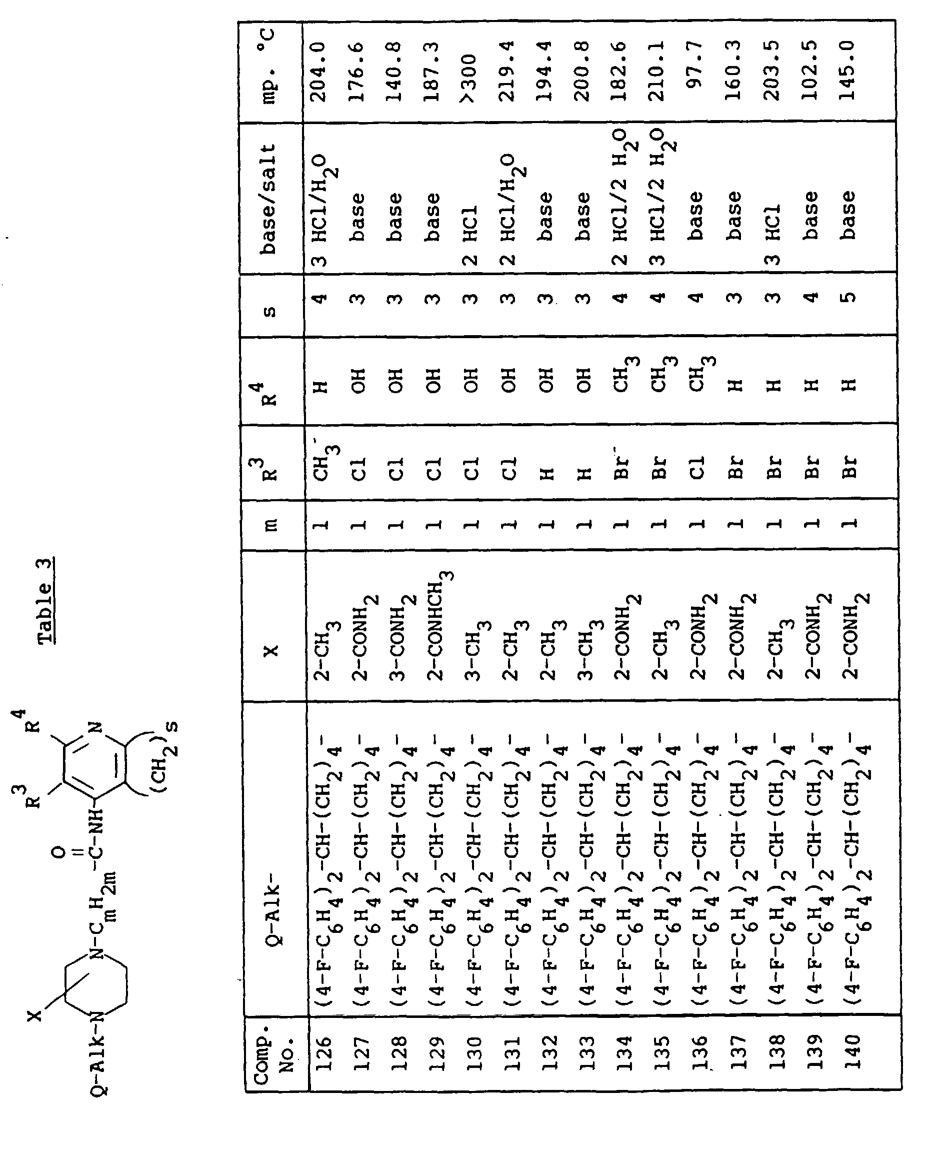

### Pharmacological Example.

The useful sleep improving properties of the compounds of formula (I) to be used in the method of the present invention can be demonstrated by the following experiment.

### Slow-wave Sleep in Dogs-Test

Fourteen adult Beagle dogs weighing 15.2 ±0.79 kg were implanted with cortical and depth electrodes. A minimum period of 4 weeks elapsed between implantation and drug studies. During this time they were adapted to the sound-attenuated and illuminated cage. The dogs' behaviour was followed by closed-circuit television.

Sixteen hours sleep recordings were made from 15.00 to 07.00 h. The first 3 hours were recorded on paper and the whole 16 hours period was analyzed by computer. Visual and computer-analysis was done on 30 sec. epochs, which were classified into wakefulness, transition to sleep, light slow wave sleep, deep slow wave sleep and rapid eye movement (REM) sleep. One cortical derivation (left frontal-occipital), the hippocampus, the electromyogram (EMG) and the electro-oculogram (EOG) were analyzed on-line by a PDP 11/23 computer. Power spectral analysis using a Fast Fourier Transformation was done on the frontal-occipital derivation each 30 sec.
The power in the frequency bands δ (0.5-3.5 Hz), θ (3.5-7.5 Hz), α (7.5-13.5 Hz) and β (13.5-25 Hz) was calculated. Additionally the power in the θ-band from the hippocampus derivation was calculated, as well as spindly activity, EMG and EOG amplitude. On the basis of these parameters, automatic sleep stage classification was done using a minimal distance approach, Electroencept. clin. Neurophysiol., 46 (1979) 33-48.

The compounds of formula (I) were given orally at the doses 0.16 and 0.63 mg/kg, just preceding the start of the recording. Table 4 illustrates the mean percent difference of slow-wave sleep with the control (equalized at 0%) based on the duration of the stage.

**Table 4:**

| mean percent difference of slow-wave sleep with the control | | |
|---|---|---|
| Comp. No. | 0.16mg/kg | 0.63mg/kg |
| 108 | 11 | 05 |
| 109 | 12 | 16 |
| 10 | - | 19 |
| 11 | - | 15 |
| 12 | 20 | 18 |
| 13 | - | 17 |
| 14 | 23 | 15 |
| 16 | - | 19 |
| 30 | - | 14 |
| 31 | - | 22 |
| 43 | 23 | - |
| 54 | 24 | - |
| 86 | 28 | - |
| 94 | 20 | - |
| 114 | 20 | - |
| 115 | 25 | - |
| 123 | 25 | - |
| 134 | 24 | - |

## Claims

1. The use for the manufacture of a medicament for improving sleep or treating sleep disorders of a compound of formula 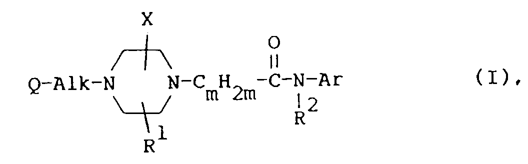 a stereochemically isomeric form or a pharmaceutically acceptable acid addition salt thereof, wherein:
R¹ is hydrogen or C₁₋₆alkyl;
X is C₁₋₆alkyl, hydroxyC₁₋₆alkyl, C₁₋₆alkyloxyC₁₋₆alkyl, aminocarbonyl, mono- and di(C₁₋₆alkyl)aminocarbonyl, carboxyl, C₁₋₆alkyloxycarbonyl, (aminocarbonyl)C₁₋₆alkyl, [mono- and di(C₁₋₆alkyl)aminocarbonyl]C₁₋₆alkyl, carboxylC₁₋₆alkyl, (C₁₋₆alkyloxycarbonyl)C₁₋₆alkyl or (hydroxyC₁₋₆alkyl)aminocarbonyl;
m is the integer 1 or 2;
R² is hydrogen or C₁₋₆alkyl;
Ar is phenyl, optionally substituted with up to 3 substituents each independently selected from the group consisting of hydroxy, C₁₋₆alkyl, C₁₋₆alkyloxy, halo, trifluoromethyl, C₁₋₆alkylcarbonyl, mono- and di(C₁₋₆alkyl)aminocarbonyl, aminocarbonyl, C₁₋₆alkyloxycarbonyl nitro, cyano, amino, amino-methyl, mono- and di(C₁₋₆alkyl)amino, (C₁₋₆alkylcarbonyl)amino, (aminocarbonyl)amino and phenylmethoxy; pyridinyl, optionally substituted with up to three substituents independently selected from halo and C₁₋₆alkyl; pyrazolyl, substituted with up to three substituents independently selected from C₁₋₆alkyl; or a radical of formula 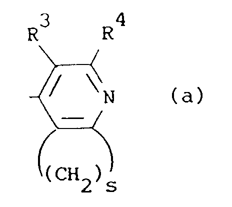 wherein R³ and R⁴ are each independently selected from the group consisting of halo, C₁₋₆alkyl, hydroxy and C₁₋₆alkyloxy and s is the integer 3, 4 or 5;
Alk is a C₁₋₆alkanediyl radical or a C₃₋₆alkenediyl radical, said C₁₋₆alkanediyl radical being optionally substituted with a hydroxy- or a C₁₋₆alkyl radical; and
Q is aryl, aryloxy, diarylmethoxy, 2,2-diarylethenyl, diarylmethylcarbonyl, arylcarbonyl, mono- and diarylaminocarbonyl, diarylmethyl or arylamino, the amino moiety in said arylamino being optionally substituted with an aryl-, an arylcarbonyl-, a C₁₋₆alkylcarbonyl-, an arylsulfonyl- or a C₁₋₆alkylsulfonyl-radical;
wherein aryl is phenyl, substituted phenyl, naphthalenyl, thienyl or pyridinyl, said substituted phenyl having from 1 to 2 substituents, each independently selected from the group consisting of halo and C₁₋₆alkyloxy.

2. The use according to claim 1 of a pharmaceutical composition containing a compound of formula (I) as defined in claim 1, and a suitable pharmaceutically acceptable carrier.

3. The use according to any of claims 1 or 2 wherein R¹ and R² are both hydrogen; m is 1; and X is C₁₋₆alkyl, hydroxyC₁₋₆alkyl, aminocarbonyl or mono- and di(C₁₋₆alkyl)aminocarbonyl.

4. The use according to claim 3 wherein Q is diarylmethoxy, 2,2-diarylethenyl, diarylaminocarbonyl, diarylmethyl or arylamino, the amino moiety in said arylamino being substituted with an aryl- or an arylcarbonyl radical; and said aryl being phenyl or substituted phenyl.

5. The use according to claim 4 wherein Q-Alk is 5,5-di(halophenyl)pentenyl or 5,5-di(halophenyl)pentyl.

6. The use according to claims 1 or 2 wherein the compound of formula (I) is 2-(aminocarbonyl)-N-(4-amino-2,6-dihalophenyl)-4-[5,5-bis(4-fluorophenyl)pentyl]-1-piperazineacetamide.

## Patentansprüche

1. Verwendung einer Verbindung der Formel 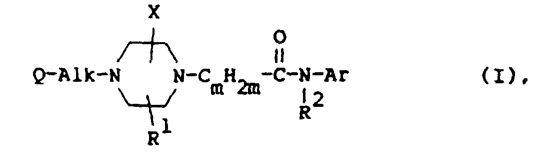 worin:
R¹ für Wasserstoff oder C₁₋₆-Alkyl steht;
X für C₁₋₆-Alkyl, Hydroxy-C₁₋₆-alkyl, C₁₋₆-AlkyloxyC₁₋₆-alkyl, Aminocarbonyl, Mono- und Di(C₁₋₆-alkyl) aminocarbonyl, Carboxyl, C₁₋₆-Alkyloxycarbonyl, Aminocarbonyl-C₁₋₆-alkyl, Mono- und Di(C₁₋₆-alkyl) aminocarbonyl-C₁₋₆-alkyl, Carboxyl-C₁₋₆-alkyl, C₁₋₆-Alkyloxycarbonyl-C₁₋₆-alkyl oder (Hydroxy-C₁₋₆-alkyl)aminocarbonyl steht;
m für die ganze Zahl 1 oder 2 steht;
R² für Wasserstoff oder C₁₋₆-Alkyl steht;
Ar für gegebenenfalls mit bis zu 3 Substituenten, die jeweils unabhängig voneinander aus der Gruppe, bestehend aus Hydroxy, C₁₋₆-Alkyl, C₁₋₆-Alkyloxy, Halogen, Trifluormethyl, C₁₋₆-Alkylcarbonyl, Mono- und Di(C₁₋₆-alkyl)aminocarbonyl,Aminocarbonyl,C₁₋₆-Alkyloxycarbonyl, Nitro, Cyano, Amino, Aminomethyl, Mono- und Di(C₁₋₆-alkyl)amino, (C₁₋₆-Alkylcarbonyl) amino, (Aminocarbonyl)amino und Phenylmethoxy, ausgewählt sind, substituiertes Phenyl; gegebenenfalls mit bis zu drei Substituenten, die unabhängig voneinander unter Halogen und C₁₋₆-Alkyl ausgewählt sind, substituiertes Pyridinyl; mit bis zu drei Substituenten, die unabhängig voneinander unter C₁₋₆-Alkyl ausgewählt sind, substituiertes Pyrazolyl; oder einen Rest der Formel 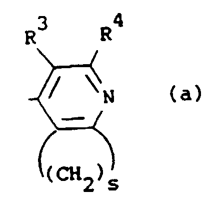 worin R³ und R⁴ jeweils unabhängig voneinander aus der Gruppe, bestehend aus Halogen, C₁₋₆-Alkyl, Hydroxy und C₁₋₆-Alkyloxy, ausgewählt sind und s die ganze Zahl 3, 4 oder 5 bedeutet, steht;
Alk für einen gegebenenfalls mit einem Hydroxy- oder einem C₁₋₆-Alkylrest substituierten C₁₋₆-Alkandiylrest oder einen C₃₋₆-Alkendiylrest steht; und
Q für Aryl, Aryloxy, Diarylmethoxy, 2,2-Diarylethenyl, Diarylmethylcarbonyl, Arylcarbonyl, Mono- oder Diarylaminocarbonyl, Diarylmethyl oder Arylamino mit gegebenenfalls durch einen Aryl-, einen Arylcarbonyl-, einen C₁₋₆-Alkylcarbonyl-, einen Arylsulfonyl- oder einen C₁₋₆-Alkylsulfonylrest substituierter Aminogruppe; worin Aryl für gegebenenfalls mit 1 bis 2 Substituenten, die jeweils unabhängig voneinander aus der Gruppe, bestehend aus Halogen und C₁₋₆-Alkyloxy ausgewählt sind, substituiertes Phenyl, Naphthalinyl, Thienyl oder Pyridinyl steht, steht,
eines ihrer stereochemisch isomeren Salze oder eines ihrer pharmazeutisch unbedenklichen Säureadditionssalze, zur Herstellung eines Einschlaf- oder Durchschlafmittels.

2. Verwendung einer pharmazeutischen Zusammensetzung nach Anspruch 1, wobei diese neben einem geeigneten pharmazeutisch unbedenklichen Träger eine Verbindung der Formel (I) gemäß Anspruch 1 enthält.

3. Verwendung nach einem der Ansprüche 1 oder 2, wobei R¹ und R² jeweils für Wasserstoff stehen; m für 1 steht; und X für C₁₋₆-Alkyl, Hydroxy-C₁₋₆-alkyl, Aminocarbonyl oder Mono- und Di(C₁₋₆-alkyl)aminocarbonyl steht.

4. Verwendung nach Anspruch 3, wobei Q für Diarylmethoxy, 2,2-Diarylethenyl, Diarylaminocarbonyl, Diarylmethyl oder Arylamino mit einer mit einem Aryl- oder Arylcarbonylrest substituierten Aminogruppe, wobei Aryl für gegebenenfalls substituiertes Phenyl steht, steht.

5. Verwendung nach Anspruch 4, wobei Q-Alk für 5,5-Di(halogenphenyl)pentenyl oder 5,5-Di(halogenphenyl)pentyl steht.

6. Verwendung nach Anspruch 1 oder 2, wobei es sich bei der Verbindung der Formel (I) um 2-Aminocarbonyl-N-(4-amino-2,6-dihalogenphenyl)-4-[5,5-bis(4-fluorphenyl)-pentyl]-1-piperazinacetamid handelt.

## Revendications

1. Utilisation, pour la fabrication d'un médicament servant à améliorer le sommeil ou à traiter les troubles du sommeil, d'un composé de formule 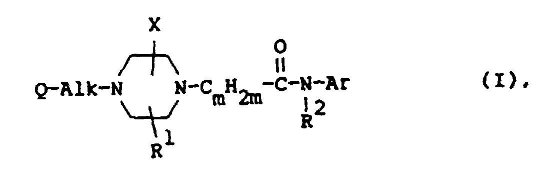 d'une forme stéréochimique isomère de ce composé ou d'un sel d'addition d'acide pharmaceutiquement acceptable de ce composé, dans laquelle
R¹ est un atome d'hydrogène ou un groupe alkyle en C₁-C₆;
X est un groupe alkyle en C₁-C₆, hydroxyalkyle en C₁-C₆, alkyl(en C₁-C₆)oxy(alkyle en C₁-C₆), aminocarbonyle, mono- et di-alkyl(en C₁-C₆)aminocarbonyle, carboxyle, alkyl (en C₁-C₆)oxycarbonyle, aminocarbonyl (alkyle en C₁-C₆), mono- et di-alkyl(en C₁-C₆)aminocarbonyl(alkyle en C₁-C₆), carboxyl(alkyle en C₁-C₆), alkyl(en C₁-C₆)oxycarbonyl(alkyle en C₁-C₆) ou hydroxyalkyl(en C₁-C₆)aminocarbonyle;
m est le nombre entier 1 ou 2;
R² est un atome d'hydrogène ou un groupe alkyle en C₁-C₆;
Ar est un groupe phényle, éventuellement substitué par au plus 3 substituants, chacun étant choisi indépendamment dans l'ensemble constitué par les groupes hydroxy, alkyle en C₁-C₆, alkyloxy en C₁-C₆, halo, trifluorométhyle, alkyl(en C₁-C₆)carbonyle, mono- et dialkyl (en C₁-C₆) aminocarbonyle, aminocarbonyle, alkyl(en C₁-C₆)oxycarbonyle, nitro, cyano, amino, aminométhyle, mono- et di-alkyl(en C₁-C₆)amino, alkyl(en C₁-C₆)carbonylamino, (aminocarbonyl)amino et phénylméthoxy; un groupe pyridinyle, éventuellement substitué par au plus 3 substituants choisis indépendamment parmi les groupes halo et alkyle en C₁-C₆; un groupe pyrazolyle, substitué par au plus 3 substituants choisis indépendamment parmi les groupes alkyle en C₁-C₆; ou un radical de formule 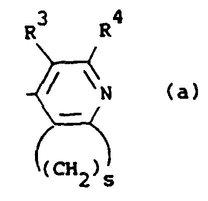 dans laquelle R³ et R⁴ sont chacun choisis indépendamment dans l'ensemble constitué par les groupes halo, alkyle en C₁-C₆, hydroxy et alkyloxy en C₁-C₆, et s est le nombre entier 3, 4 ou 5;
Alk est un radical alcane(en C₁-C₆)diyle ou un radical alcène(en C₃-C₆)diyle, ledit radical alcane(en C₁-C₆)diyle étant éventuellement substitué par un radical hydroxy ou alkyle en C₁-C₆; et
Q est un groupe aryle, aryloxy, diarylméthoxy, 2,2-diaryléthényle, diarylméthylcarbonyle, arylcarbonyle, mono- et di-arylaminocarbonyle, diarylméthyle ou arylamino, le groupement amino dans ledit groupe arylamino étant éventuellement substitué par un radical aryle, arylcarbonyle, alkyl(en C₁-C₆)carbonyle, arylsulfonyle ou alkyl(en C₁-C₆)sulfonyle;
où le groupe aryle est un groupe phényle, phényle substitué, naphtalényle, thiényle ou pyridinyle, ledit groupe phényle substitué comportant de 1 à 2 substituants, chacun étant choisi indépendamment dans l'ensemble constitué par les groupes halo et alkyloxy en C₁-C₆.

2. Utilisation selon la revendication 1 d'une composition pharmaceutique contenant un composé de formule (I) telle que définie dans la revendication 1 et un véhicule pharmaceutiquement acceptable convenable.

3. Utilisation selon l'une quelconque des revendications 1 et 2, dans laquelle R¹ et R² sont tous deux des atomes d'hydrogène; m est égal à 1; et X est un groupe alkyle en C₁-C₆, hydroxyalkyle en C₁-C₆, aminocarbonyle ou mono- et di-alkyl(en C₁-C₆)aminocarbonyle.

4. Utilisation selon la revendication 3, dans laquelle Q est un groupe diarylméthoxy, 2,2-diaryléthényle, diarylaminocarbonyle, diarylméthyle ou arylamino, le groupement amino dans ledit groupe arylamino étant substitué par un radical aryle ou arylcarbonyle; et ledit groupe aryle étant un groupe phényle ou phényle substitué.

5. Utilisation selon la revendication 4, dans laquelle Q-Alk est un groupe 5,5-di(halophényl)pentényle ou 5,5-di(halophényl)pentyle.

6. Utilisation selon la revendication 1 ou 2, dans laquelle le composé de formule (I) est le 2-(aminocarbonyl)-N-(4-amino-2,6-dihalophényl)-4,[5,5-bis(4-fluorophényl)pentyl]-1-pipérazineacétamide.
